Europäisches Patentamt

(19) European Patent Office

Office européen des brevets

(11) Veröffentlichungsnummer: **0 216 009**
**B1**

# (12) EUROPÄISCHE PATENTSCHRIFT

(45) Veröffentlichungstag der Patentschrift:
05.10.88

(51) Int. Cl.⁴: **C 07 C 15/24,** C 07 C 7/14

(21) Anmeldenummer: **86107031.6**

(22) Anmeldetag: **23.05.86**

(54) Verfahren zur Gewinnung von 2,6-Dialkylisomeren des Naphthalins aus dem Isomerengemisch derselben.

(30) Priorität: **04.09.85 DE 3531559**

(43) Veröffentlichungstag der Anmeldung:
**01.04.87 Patentblatt 87/14**

(45) Bekanntmachung des Hinweises auf die Patenterteilung:
**05.10.88 Patentblatt 88/40**

(84) Benannte Vertragsstaaten:
**CH DE FR GB LI NL**

(56) Entgegenhaltungen:
**US - A - 3 173 960**
**US - A - 3 202 726**

**CHEMICAL ABSTRACTS, vol. 85, no. 1, 5th July 1976,
page 434, abstract no. 5423e, Columbus, Ohio, US; &
JP-A-75 17 986 (TEIJIN LTD.) 25-06-1975**

(73) Patentinhaber: **RÜTGERSWERKE
AKTIENGESELLSCHAFT, Mainzer Landstrasse 217,
D-6000 Frankfurt am Main 1 (DE)**

(72) Erfinder: **Höltmann, Wilhelm, Dr., Dorffeldstrasse 9a,
D-4400 Münster (DE)**
Erfinder: **Zellerhoff, Robert, Dr., Möllenkampweg 7,
D-4236 Hamminkeln 4 (DE)**
Erfinder: **Oberkobusch, Rudolf, Dr., Varziner Strasse 15,
D-4100 Duisburg 12 (DE)**
Erfinder: **Stäglich, Peter, Dr., Mommsenstrasse 12,
D-4152 Kempen 1 (DE)**
Erfinder: **Charpey, Bernhard, Parsevalstrasse 54,
D-4330 Mülheim (Ruhr) (DE)**

## Beschreibung

Verfahren zur Gewinnung von 2,6-Dialkylisomeren des Naphtalins aus dem Isomerengemisch derselben.

Die Erfindung betrifft einen Verfahrensschritt bei der Gewinnung von technisch reinen 2,6-Dialkylnaphthalinen mit einem Gehalt des 2,6-Isomeren von mindestens 95%.

2,6-Dialkylnaphthaline sind u.a. Ausgangsprodukte für die Herstellung von 2,6-Naphthalindicarbonsäure, der Säurekomponente thermisch besonders stabiler Kondensationspolymerer. Gerade für diese Verwendung ist ein möglichst isomerenreines Produkt erforderlich.

2,6-Dialkylnaphthaline fallen bei der Alkylierung von Naphthalin im Gemisch mit zahlreichen Isomeren an, wobei der Gehalt an 2,6-Dialkylnaphthalin in diesem Isomerengemisch je nach Alkylierungsverfahren zwischen 5 und 50% liegt. Um durch gängige Trenn- und Reinigungsverfahren zu einem technisch reinen Produkt zu gelangen, muss 2,6-Dialkylnaphthalin zuvor auf einen Gehalt von etwa 80 bis 90% angereichert werden. Insbesondere muss darauf geachtet werden, dass dabei keine Anreicherung des 1,5-Dialkylnaphthalins erfolgt, denn bei Gehalten von mehr als 5% dieses Isomeren ist eine wirtschaftliche Isolierung des reinen 2,6-Dialkylnaphthalins nicht mehr möglich. Wünschenswert ist deshalb eine möglichst weitgehende und frühzeitige Abtrennung des 1,5-Dialkylnaphthalins. Ebenso müssen insbesondere die 2,7-, 1,3- und 1,6-Isomere abgetrennt werden. Diese Abtrennung ist mit gängigen Verfahren nicht ohne weiteres möglich, so dass eine wirtschaftliche Gewinnung von technisch reinen 2,6-Dialkylnaphthalinen nicht gegeben ist.

Es ist daher Aufgabe der vorliegenden Erfindung, ein einfaches, wirtschaftliches Verfahren zur Gewinnung eines technisch reinen 2,6-Dialkylnaphthalins aus einem Isomerengemisch bereitzustellen, bei dem insbesondere die 1,5-Isomeren, wie auch die 1,3-, 1,6- und 2,7-Isomeren möglichst weitgehend abgetrennt werden.

Die Lösung der Aufgabe erfolgt durch ein Verfahren gemäss der Ansprüche 1 bis 9.

Bei üblichen Alkylierungs- und Transalkylierungsverfahren anfallende Dialkylnaphthalinfraktionen haben Isomerenverteilungen, die in der folgenden Grössenordnung liegen:

| | | |
|---|---|---|
| 1,3-Dialkylnaphthalin | » | 5-34% |
| 1,5- | » | 1-12% |
| 1,6- | » | 1-15% |
| 1,7- | » | 1-29% |
| 2,6- | » | 12-36% |
| 2,7- | » | 9-35% |

Definierte Gemische sind in den Beispielen angegeben. Erfindungsgemäss aufarbeitbare Dialkylnaphthaline sind insgesamt Methyl-n-propyl-, Methyl-isopropyl-, Methyl-n-butyl, Methyl-isobutyl-, Ethyl-isopropyl-, Ethyl-n-butyl-, Di-n-propyl-, Di-isopropyl-, Methyl-amyl-, Ethyl-amyl-, Propyl-amyl-, Di-n-butyl- oder Diisobutyl-naphthaline, vorzugsweise die für die Herstellung thermisch besonders stabiler Polykondensate technisch interessanten Methylisopropyl- und Diisopropylnaphthaline.

Wird eine derartige beispielhafte Diisopropylnaphthalinfraktion der für Isomerentrennungen üblichen stufenweisen Kältekristallisation mit Animpfen der Fraktion mit den jeweiligen Reinprodukten unterworfen, so können nach 96 h bei –15°C 24% Kristallgut abfiltriert werden, in dem die 1,3- und 1,7-Isomere auf etwa 70% angereichert sind. Nach weiteren 7 d bei –15°C werden zusätzlich 6% Kristallgut gewonnen, das neben 67% 2,6- auch 1% 1,5- und 26% 1,3-Diisopropylnaphthalin enthält. Aus dieser Fraktion lässt sich durch übliche Verfahren der Lösungsmittel- oder Schmelzkristallisation wirtschaftlich kein reines 2,6-Diisopropylnaphthalin isolieren.

Ausserdem scheidet eine technische Gewinnung aufgrund der langen Kristallisationszeiten aus. Eine Absenkung der Kühlendtemperatur auf –25 oder –30°C erhöht zwar die Kristallausbeute auf 8-12%, jedoch enthält die gewünschte Kristallfraktion neben 77% des 2,6- auch 12% des 1,5-Isomeren. Die Trennung der beiden Isomeren in dieser Zusammensetzung ist nach dem üblichen Trennverfahren in einer technischen Produktion nicht möglich, da sich hierbei das 1,5-Isomere anreichert.

Es wurde bei diesen Versuchen auch gefunden, dass die Ausfällung von 2,6-Diisopropylnaphthalin durch Schütteln oder wiederholtes kurzzeitiges Rühren beschleunigt wird. Jedoch zeigte sich, dass dabei der Anteil an 1,5-Diisopropylnaphthalin im Kristallgut ebenfalls auf Werte über 12% steigt.

Eine Verkürzung der Kristallisationszeit bei gleichzeitiger guter Ausbeute erbrachte die aus C.A. 85:5423e für die Abtrennung des 2,6- von 2,7-Diisopropylnaphthalin bekannte Ausfällung mit Hilfe von Alkohol.

Es zeigte sich, dass aus Mischungen einer Diisopropylnaphthalinfraktion mit einem polaren Lösungsmittel im Verhältnis 1:0,5 bis 4,0 beim Abkühlen zuerst eine mit 2,6-Diisopropylnaphthalin angereicherte Fraktion ausfällt und dass auch bei einer Temperatur von –10 bis –20°C innerhalb von 24 bis 48 h eine wirtschaftlich vertretbare Ausbeute von 8 bis 12% an Rohkristallisat erzielt wird. Jedoch reichert sich auch bei diesem Verfahren das 1,5-Isomere im Kristallgut an. Beim Durchführen dieser Fällungen unter gelegentlichem Umrühren werden Gehalte von 12 bis 30% 1,5-Diisopropylnaphthalin im Kristallgut erhalten.

Es ist daher überraschend, dass bei der Kältekristallisation von Dialkylnaphthalinfraktionen mit einem polaren Lösungsmittel unter konstantem Rühren im Temperaturbereich von Raumtemperatur bis –30°C, abhängig vom jeweiligen 2,6-Isomerengehalt der Einsatzfraktion, ein Kristallisat anfällt mit Gehalten von 85 bis 95% an 2,6-Dialkylnaphthalin und bis maximal 2% an 1,5-Dialkylnaphthalin, das nach einmaligem Umfällen ein technisch reines 2,6-Dialkylnaphthalin mit Gehalten von 95-99% ergibt.

Als polare Lösungsmittel können niedrige Alkohole wie etwa Ethanol, Propanol oder Isopropanol oder auch Ketone wie z.B. Aceton, Methylethylketon oder Methylisobutylketon oder aber auch niedere chlorierte Kohlenwasserstoffe wie Choloroform oder Methylchlorid u.a. verwendet werden. Die eingesetzte Lösungsmittelmenge liegt im Bereich von 0,5 bis 4,0, bevorzugt bei 1 bis 2 Gewichtsteilen je Gewichtsteil Dialkylnaphthalinfraktion.

Die Lösung aus Dialkylnaphthalinfraktion und Lösungsmittel wird unter ständigem Rühren abgekühlt. Wichtig dabei ist, dass die Lösung bzw. Dispersion in ständiger Bewegung gehalten wird, um die optimale Bildung der gewünschten Kristallisationskeime zu gewährleisten.

Jedoch soll die Rührbewegung nicht wesentlich über der kritischen Drehzahl des Rührers liegen, da dann die bevorzugte Ausfällung des 2,6-Isomeren beeinträchtigt wird. Die optimale Rührbewegung liegt im Bereich der kritischen Drehzahl des Rührers. Andererseits ist die Bewegung des Gemisches erst ab Beginn der Kristallisation erforderlich. Da dies aber kein fester Punkt ist und sich andererseits durch die Bewegung des Gemisches ein früherer Kristallisationsbeginn einstellt, ist es empfehlenswert, das Gemisch vom Beginn der Abkühlphase an zu rühren. Die Abkühlung der Mischung kann kontinuierlich oder stufenweise erfolgen, wobei die optimalen Ergebnisse bei einer kontinuierlichen Abkühlung erhalten werden mit Abkühlraten im Bereich von 2 bis 15°C/h, vorzugsweise von 2 bis 5°C/h.

Bei Dialkylnaphthalinfraktionen mit einem Gehalt an 2,6-Isomeren unter 30% liegt die Auskühltemperatur vorteilhafterweise im Bereich von 0 bis –20°C, bei höheren Gehalten im Bereich 20 bis 0°C. Zur weiteren Beschleunigung der Ausfällung ist es von Vorteil, die vorgekühlte Mischung vor Beginn der Kristallisation mit reinem 2,6-Dialkylnaphthalin anzuimpfen.

Das ausgefallene Kristallgut wird mechanisch, vorzugsweise mittels einer Zentrifuge, von der Mutterlauge getrennt. Die Hauptkomponente 2,6-Dialkylnaphthalin kann danach leicht durch übliche Kristallisationsverfahren zum hochreinen Produkt (99%) gereinigt werden.

Das erfindungsgemässe Verfahren erlaubt es prinzipiell, 2,6-Dialkylnaphthalin aus Isomerengemischen zu gewinnen, die einen 2,6-Isomerengehalt von 10% und höher haben.

*Beispiele*

Alle Prozentangaben im Beispiel und in der Beschreibung sind Angaben in Gewichtsprozent.

*Beispiel 1*

5000 Gew.-Teile eines Isomerengemisches der folgenden Zusammensetzung der Hauptisomeren

| | | |
|---|---|---|
| 1,3-Diisopropylnaphthalin | 16% |
| 1,5- | » | 5% |
| 1,7- | » | 17% |
| 2,6- | » | 20% |
| 2,7- | » | 17% |

werden bei Raumtemperatur mit 2500 Gew.-Teilen Ethanol (99%) vermischt und unter ständigem Rühren innerhalb von 4 h auf –10°C gekühlt.

Danach wird das Gemisch mit 0,2 Gew.-Teilen reinem 2,6-Diisopropylnaphthalin angeimpft und unter ständigem Rühren innerhalb weiterer 3 h auf –20°C abgekühlt. Die Rührbewegung ist dabei so intensiv, dass sich kein Kristallgut im Kristallisationsgerät absetzt.

Nach Filtration fallen 330 Gew.-Teile eines Rohkristallisats an mit der folgenden Zusammensetzung:

| | | |
|---|---|---|
| 2,6-Diisopropylnaphthalin | 86% |
| 2,7- | » | 3% |
| 1,3- | » | 3% |
| 1,5- | » | 2% |
| Restisomere | | 6% |

Von der Mutterlauge wird das Ethanol abdestiliert und in die Kristallisationsstufe zurückgeführt. Das zurückbleibende Isomerengemisch wird einem anderen Verwendungszweck zugeführt.

Das Rohkristallisat wird mittels fraktionierter Schmelz- oder Lösungsmittelkristallisation weiter gereinigt und liefert reines 2,6-Diisopropylnaphthalin.

*Beispiel 2*

2500 Gew.-Teile eines Isomerengemisches wie in Beispiel 1 werden bei Raumtemperatur mit 3750 Gew.-Teilen Isopropanol vermischt und unter ständigem Rühren innerhalb von 3,5 h auf –11°C abgekühlt. Danach wird das Gemisch mit 0,2 Gew.-Teilen reinem 1,6-Diisopropylnaphthalin angeimpft und unter ständigem Rühren innerhalb weiterer 3 h auf –20°C abgekühlt. Nach weiteren 2 h bei –20°C erhält man nach Abtrennen des Kristallgutes 151 Gew.-Teile eines Rohkristallisates folgender Zusammensetzung:

| | | |
|---|---|---|
| 89% | 2,6-Diisopropylnaphthalin | |
| 5% | 2,7- | » |
| 1% | 1,5- | » |
| 1% | 1,3- | » |
| 4% | Restisomere | |

Vom verbleibenden Filtrat wird das Isopropanol durch Destillation abgetrennt und das Restisomerengemisch einer anderen Verwendung zugeführt.

Das Rohkristallisat wird durch Schmelz- oder Lösungsmittelkristallisation weiter gereinigt und liefert reines 2,6-Diisopropylnaphthalin.

*Beispiel 3*

290 Gew.-Teile eines Isopropylmethylnaphthalinisomerengemischs mit einem Gehalt von 46% an 2-Methyl-6-isopropylnaphthalin, 39% 2-Methyl-7-isopropylnaphthalin und 15% an Restisomeren werden analog Beispiel 1 bei Raumtemperatur mit 435 Gew.-Teilen Ethanol (99%ig) vermischt und unter ständigem Rühren innerhalb von 8 h auf –10°C abgekühlt. Danach versetzt man mit 0,2 Gew.-Teilen reinem 2-Methyl-6-isopropylnaphthalin und kühlt innerhalb weiterer 5 h unter ständigem Rühren auf –20°C ab. Dabei fallen 32 Gew.-Teile eines Rohkristallisats an, die durch Filtration von Ethanol abgetrennt werden. Die Zusammensetzung des Rohkristallisats beträgt 95% an 2-Methyl-6-isopropylnaphthalin, 3% an 2-Methyl-7-isopropylnaphthalin und 2% Restisomere. Das Filtrat wird durch Destillation von Ethanol befreit und das verbleibende Isomerengemisch einer anderen Verwendung zugeführt. Das Rohkristallisat wird mittels fraktionierter Schmelz- oder Lösungskristallisation weiter gereinigt und liefert reines 2-Methyl-6-isopropylnaphthalin.

*Vergleichsbeispiel 1*

175 Gew.-Teile eines Isomerengemisches mit der folgenden Zusammensetzuung der Hauptisomeren

| | | |
|---|---|---|
| 1,3-Diisopropylnaphthalin | 15% |
| 1,5- | » | 6% |
| 1,7- | » | 17% |
| 2,6- | » | 20% |
| 2,7- | » | 17% |

werden auf –15°C abgekühlt und 96 h bei dieser Temperatur ausgekühlt. Das ausgefallene Kristallgut wird abgetrennt und man erhält 41,3 Gew.-Teile eines Rohkristallisates mt einem Gehalt von 71% 1,3- und 1,7-Diisopropylnaphthalin, 27% 2,6-Diisopropylnaphthalin und 2% Restisomere. Nach weiteren 168 h bei 15°C erhält man weitere 10,7 Gew.-Teile an Kristallgut mit der Zusammensetzung: 67% 2,6-Diisopropylnaphthalin, 5% 2,7-Diisopropylnaphthalin, 26% 1,3-Diisopropylnaphthalin, 2% 1,5-Diisopropylnaphthalin.

*Vergleichsbeispiel 2*

600 Gew.-Teile eines Isomerengemisches der gleichen Zusammensetzung wie in Vergleichsbeispiel 1 werden unter Rühren auf –25°C gekühlt. Nach 97 h bei 25°C erhält man 19,4 Gew.-Teile eines Rohkristallisates folgender Zusammensetzung: 71% 2,6-Diisopropylnaphthalin, 6% 2,7-Diisopropylnaphthalin, 23% 1,5-Diisopropylnaphthalin. Nach weiteren 96 h bei –25°C erhält man weitere 21,3 g Rohkristallisat mit im wesentlichen 44% 2,6-Diisopropylnaphthalin und 30% 1,5-Diisopropylnaphthalin.

*Vergleichsbeispiel 3*

2000 Gew.-Teile eines Isomerengemisches wie in Vergleichsbeispiel 1 werden mit 5000 Gew.-Teilen Ethanol (99%ig) vermischt und 120 h lang bei –30°C ausgekühlt. Man erhält 357 Gew.-Teile eines Rohkristallisates mit im wesentlichen 61% 2,6- 19% 1,5- und 8% 2,7-Diisopropylnaphthalin.

*Vergleichsbeispiel 4*

2500 Gew.-Teile eines Isomerengemisches wie in Vergleichsbeispiel 1 werden mt 3750 Gew.-Teilen Ethanol (99%ig) vermischt und auf –15°C abgekühlt. Nach 25,7 h Intervallrühren alle 30 min für jeweils 5 min erhält man 146 Gew.-Teile eines Rohkristallisates folgender Zusammensetzung:

| | | |
|---|---|---|
| 2,6-Diisopropylnaphthalin | 77% |
| 1,5- | » | 19% |
| 2,7- | » | 2% |
| Restisomere | | 2% |

**Patentansprüche**

1. Verfahren zur Gewinnung von 2,6-Dialkylnaphthalinen aus der Gruppe der Methyl-n-propyl-, Methyl-isopropyl-, Methyl-n-butyl-, Methyl-isobutyl-, Ethyl-isopropyl-, Ethyl-n-butyl-, Di-n-propyl-, Di-isopropyl-, Methyl-amyl-, Ethyl-amyl-, Propyl-amyl-, Di-n-butyl- oder Diisopropylnaphthaline aus ihren Isomerengemischen durch Kristallisation aus einer Lösung in einem polaren Lösungsmittel, dadurch gekennzeichnet, dass die Ausfällung des 2,6-Dialkylnaphthalins auch in Gegenwart anderer hochschmelzender Isomeren unter ständigem Rühren durch Abkühlen auf eine Temperatur im Bereich von +25 bis –30°C erfolgt.

2. Verfahren nach Anspruch 1, dadurch gekennzeichnet, dass die Rührbewegung im Bereich der kritischen Drehzahl liegt.

3. Verfahren nach Anspruch 1, dadurch gekennzeichnet, dass das Verhältnis Isomerengemisch zu Lösungsmittel im Bereich von 1 zu 0,5 - 4,0 liegt.

4. Verfahren nach Anspruch 1, dadurch gekennzeichnet, dass das Verhältinis Isomerengemisch zu Lösungsmittel im Bereich von 1 - 2 liegt.

5. Verfahren nach Anspruch 1, dadurch gekennzeichnet, dass die Abkühlrate 2 bis 15°C pro Stunde beträgt.

6. Verfahren nach Anspruch 1, dadurch gekennzeichnet, dass das gelöste Isomerengemisch unter Rühren mit einer Abkühlrate von 2 bis 15°C pro Stunde abgekühlt wird, wobei vor Beginn der Kristallisation mit reinem 2,6-Dialkylnaphthalin angeimpft wird.

7. Verfahren nach Anspruch 1 bis 6, dadurch gekennzeichnet, dass aus Dialkylnaphthalingemischen mit mehr als 10 Gew.-% an 2,6-Dialkylnaphthalin das 2,6-Isomere gewonnen wird.

8. Verfahren nach Anspruch 1 bis 7, dadurch gekennzeichnet, dass 2,6-Diisopropylnaphthalin in Gegenwart von 1,5-Diisopropylnaphthalin gewonnen wird.

9. Verfahren nach Anspruch 1 bis 7, dadurch gekennzeichnet, dass 2-Isopropyl-6-methylnaphthalin gewonnen wird.

**Claims**

1. A process for the recovery of 2.6-dialkyl naphthalenes from the group of methyl-n-propyl, methyl-isopropyl, methyl-n-butyl, methyl-isobutyl, ethyl-isopropyl, ethyl-n-butyl, di-n-propyl, di-isopropyl, methyl-amyl, ethyl-amyl, propyl-amyl, di-n-butyl or diisobutyl naphthalenes from mixtures of the isomers thereof by crystallization from a solution in a polar solvent, characterized in that the precipitation of the 2.6-dialkyl naphthalene also in the presence of other high-melting isomers is performed during constant stirring by cooling to a temperature in the range of +25 to –30°C.

2. A process according to Claim 1, characterized in that the stirring movement is in the range of the critical speed.

3. A process according to Claim 1, characterized in that the ratio of the isomer mixture to the solvent is in the range of 1 tq 0.5 - 4.0.

4. A process according to Claim 1, characterized in that the ratio of the isomer mixture to the solvent is in the range of 1 - 2.

5. A process according to Claim 1, characterized in that the cooling rate amounts to 2 to 15°C per hour.

6. A process according to Claim 1, characterized in that the dissolved isomer mixture is cooled during stirring at a cooling rate of 2 to 15°C per hour, seeding with pure 2.6-dialkyl naphthalene taking place before the beginning of crystallization.

7. A process according to Claim 1 to 6, characterized in that the 2.6-isomer is recovered from dialkyl naphthalene mixture with more than 10% by weight of 2.6-dialkyl naphthalene.

8. A process according to Claims 1 to 7, characterized in that 2.6-diisopropyl naphthalene is recovered in the presence of 1.5-diisopropyl naphthalene.

9. A process according to Claims 1 to 7, characterized in that 2-isopropyl-6-methyl naphthalene is recovered.

## Revendications

1. Procédé pour l'obtention de 2,6-dialkylnaphthalènes du groupe des méthyl-n-propyl-, méthyl-isopropyl-, méthyl-n-butyl-, méthyl-isobutyl-, éthyl-isopropyl-, éthyl-n-butyl-, di-n-propyl-, di-isopropyl-, méthyl-amyl-, éthyl-amyl-, propyl-amyl-, di-n-butyl-ou diisobutylnaphthalènes à partir de leurs mélanges d'isomères par cristallisation dans une solution, caractérisé en ce que la précipitation préférée du 2,6-dialylnaphthalène s'effectue également en présence d'autres isomères à point de fusion élevé sous agitation constante par refroidissement jusqu'à une température dans le domaine de +25 à −30°C.

2. Procédé selon la revendication 1, caractérisé en ce que l'agitation se situe dans le domaine de la vitesse de rotation critique.

3. Procédé selon la revendication 1, caractérisé en ce que le rapport entre le mélange d'isomères et le solvant se situe dans le domaine de 1 à 0,5 - 4,0.

4. Procédé selon la revendication 1, caractérisé en ce que le rapport entre le mélange d'isomères et le solvant se situe dans le domaine de 1 - 2.

5. Procédé selon la revendication 1, caractérisé en ce que le gradient de refroidissement est de 2 à 15°C par heure.

6. Procédé selon la revendication 1, caractérisé en ce que le mélange d'isomères dissous est refroidi sous agitation avec un gradient de refroidissement de 2 à 15°C par heure, du 2,6-dialkylnaphthalène pur ayant été inoculé avant le début de la cristallisation.

7. Procédé selon la revendication 1 à 6, caractérisé en ce qu'à partir de mélanges de dialkylnaphthalènes comportant plus de 10% en poids de 2,6-dialkylnaphthalène, on obtient l'isomère 2,6.

8. Procédé selon la revendication 1 à 7, caractérisé en ce que le 2,6-diisopropylnaphthalène est obtenu en présence de 1,5-diisopropylnaphthalène.

9. Procédé selon la revendication 1 à 7, caractérisé en ce que l'on obtient du 2-isopropyl-6-méthyl-naphthalène.